(19) Europäisches Patentamt / European Patent Office / Office européen des brevets

(11) **EP 4 471 787 A1**

(12) **EUROPEAN PATENT APPLICATION**

(43) Date of publication:
**04.12.2024 Bulletin 2024/49**

(21) Application number: **23305847.8**

(22) Date of filing: **29.05.2023**

(51) International Patent Classification (IPC):
*G16B 40/00* *(2019.01)*

(52) Cooperative Patent Classification (CPC):
**G16B 40/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **Metafora Biosystems**
  **75014 Paris (FR)**
• **Université Paris-Saclay**
  **91190 Gif sur Yvette (FR)**
• **Centre National de la Recherche Scientifique**
  **75016 Paris (FR)**

(72) Inventors:
• **DUSSAP, Bastien**
  **91940 les Ulys (FR)**
• **BLANCHARD, Gilles**
  **72000 Le Mans (FR)**
• **LABARTHE, Baptiste**
  **75009 Paris (FR)**
• **CHERIEF-ABDELLATIF, Badr-Eddine**
  **75009 Paris (FR)**

(74) Representative: **Icosa**
  **83 avenue Denfert-Rochereau**
  **75014 Paris (FR)**

(54) **METHOD FOR DETERMINING PROPORTIONS OF POPULATIONS IN AN ENSEMBLE OF BIOLOGICAL OBJECTS**

(57) The present invention relates to a computer-implemented method (100) for determining proportions $\alpha_i$ of C reference populations ($P_i$) in an ensemble of biological objects being chosen among cells, vesicles of cellular origin, acellular microorganisms, and/or biofunctionalized materials.

FIG. 2

EP 4 471 787 A1

## Description

### FIELD OF INVENTION

The present invention relates to the field of cytometric data analysis, including single cell data analysis.

### BACKGROUND OF INVENTION

The emergence of protein sequencing, and then DNA sequencing, and the development of automatic sequencers have revolutionized biology. The classical descriptive and reductionist approach (one gene, one messenger RNA, one protein) has been replaced by a more global understanding of biological systems based on the analysis of sets of biological elements ("-omes"). The basic idea associated with "omics" approaches is to apprehend the complexity of living organisms as a whole, using methodologies that are the least restrictive possible in terms of description.

Such approaches mainly include: genomics (study of genes), transcriptomics (analysis of gene expression and its regulation), proteomics (study of proteins), metabolomics (analysis of metabolites) and metabonomics (study of metabolic profiles *in vivo*).

Genomics is divided into two branches: structural genomics, which relates to the sequencing of the entire genome, and functional genomics, which aims to determine the function and expression of the sequenced genes. In functional genomics, techniques are applied to a large number of genes in parallel: for example, the phenotype of mutants for a whole family of genes, or the expression of all the genes of an entire organism, can thus be analyzed.

Transcriptomics is the study of all the messenger RNAs produced during the genetic transcription process. It is based on the quantification of all of these messenger RNAs, which provides a relative indication of the transcription rate of different genes under given conditions.

Proteomics is the analysis of all the proteins of an organelle, a cell, a tissue, an organ or an organism under given conditions. Proteomics attempts to identify in a global way the proteins extracted from a cell culture, a tissue or a biological fluid, their localization in the cellular compartments, their possible post-translational modifications, as well as their quantity. It makes it possible to quantify the variations in their level of expression, for example as a function of time, of their environment, of their state of development, of their physiological and pathological state, of the species of origin, etc. It also studies the interactions that proteins have with other proteins, with DNA or RNA, or with other substances.

Metabolomics studies all metabolites (sugars, amino acids, fatty acids, etc.), such as metabolic substrates, intermediates, products, as well as hormones, other signaling molecules and secondary metabolites, present in a cell, an organ, an organism.

Metabonomics consists of monitoring metabolic profiles *in vivo,* which makes it possible to provide information on the toxicity of drugs, on pathological processes, and on the function of genes.

Cytomics corresponds to the analysis of the cytome, the set of cellular constituents, in particular morphological, antigenic and functional, which make it possible to define or model the state and the functioning of a cell at a given time. The analysis of the cytome makes it possible to describe the structural and functional heterogeneity of the various cells of an organism.

The preceding approaches make it possible to obtain a great amount of information on the cellular and/or tissue response to an *in vitro* or *in vivo* exposure. They can in particular be useful for highlighting and identifying new biomarkers (of diagnosis, susceptibility, prognosis, exposure, effect), generating new knowledge on the mechanistic level (modes of action), or further develop new efficacy or predictive toxicology tools to help identify new therapeutic targets or new candidate drugs or candidate vaccines.

The automation of sequencing techniques and the development of high-throughput techniques, in particular made possible by the emergence of specialized technological platforms, have allowed the industrialization of data production and the simultaneous analysis of a large number of parameters.

The result is a very large amount of data to be processed, analyzed, visualized and interpreted in the most informative way possible in order to extract the maximum amount of information on the biological process or on the biological system studied.

From the biostatistic point of view, the data obtained by "omics" approaches relate to very numerous variables that should be analyzed jointly. For example, transcriptomic analyzes make it possible to simultaneously study the expression of several thousand genes.

It is therefore desirable to have powerful biostatistical and bioinformatics means for processing, analyzing and interpreting the mass of data generated by "omics" approaches.

There are many techniques for acquiring "omics" data, such as, as non-limiting examples, mass spectrometry, chromatography, sequencing, spectral cytometry, mass cytometry, flow cytometry, etc.

Flow cytometry is a technique for analyzing a biological fluid comprising a collection of biological objects, such as cells, vesicles or particles, in suspension. Flow cytometry is an essential analytical technique for the identification and characterization of populations of cells, vesicles or particles.

Besides, individual cell heterogeneity within a cell population can be critical to its specific function and fate. Cell-to-cell variations, for instance in RNA transcripts or protein expression, may be key in research and development studies in the field of cancer, neurobiology, stem cell biology, immunology and developmental biology. Conventional cell-based assays mainly analyze the average responses from a population of cells, without providing individual cell phenotypes. To better understand cell-to-cell differences, detailed information may be provided by single cell analyses.

Single-cell analysis is the study of genomics, transcriptomics, proteomics, metabolomics and cell-cell interactions at the single cell level. Analyzing a single cell makes it possible to discover mechanisms not seen when studying a cell population. High-throughput and multiparameter approaches may be combined in single cell analysis in order to reflect cell-to-cell variability and heterogeneous differences in the individual cells.

Technologies such as fluorescence-activated cell sorting (FACS) allow the precise isolation of selected single cells from complex samples, while high throughput single cell partitioning technologies, enable the simultaneous molecular analysis of hundreds or thousands of single unsorted cells. This is particularly useful for the analysis of transcriptome variation in genotypically identical cells, allowing the definition of otherwise undetectable cell subtypes. Thus, flow cytometry can address the issue of cellular processes and intercellular interactions because cytometry provides a single-cell view of more complex cellular conglomerates, such as tissues in multicellular organisms or communities in the case of single-cell bacteria, yeasts, algae, etc.

The development of new technologies nowadays enables to analyze the genome and transcriptome of single cells, as well as to quantify their proteome and metabolome. Single-cell RNA sequencing (scRNA-seq) technology has become a state-of-the-art approach for unravelling the heterogeneity and complexity of RNA transcripts within individual cells, as well as revealing the composition of different cell types and functions within tissues, organs or organisms. In situ sequencing and fluorescence in situ hybridization (FISH) do not require that cells be isolated and may be used for analysis of tissues. Other technologies enable quantifying thousands of proteins across hundreds of single cells. For example, mass spectrometry techniques have become important analytical tools for proteomic and metabolomic analysis of single cells.

Individual cells differentially use their genes, RNA, and proteins across tissue types. Spatial biology designates a series of techniques providing detailed cellular information according to the positional context of cells in a tissue. Thus, spatial biology studies the cell types, their location in tissues, their biomarker co-expression patterns, and how cells organize, interact and influence the tissue microenvironment.

Spatial transcriptomics is a technology that allows measuring all the gene activity in a tissue sample and spatially map where the activity is occurring. It combines unbiased, high-throughput total mRNA analysis for intact tissue sections with morphological context. Spatial proteomics combines microscopy with detection of multiple proteins, usually through the use of specific antibodies. It allows the integration of morphological data and protein expression data at the single-cell level. Detection methods may be based for example on mass spectrometry, on chromogenic staining, or on oligonucleotide barcodes.

Among the various parameters that may be analyzed in a set of data obtained by flow cytometry or other assays, and corresponding to a particular ensemble of biological objects, the quantification of the different populations of biological objects contained in this ensemble is of particular interest. The quantification of the different populations of biological objects contained in an ensemble of analyzed biological objects is generally one of the first assessed parameters. Indeed, the quantification of the different populations of biological objects may for instance provide information on the validity of the data obtained during a specific assay, or on the reproducibility of this kind of assay.

It is therefore desirable to have powerful biostatistical and bioinformatics means and methods for rapidly determining quantification of different populations of biological objects for which data have been obtained by an assay. These methods must be easy to implement and robust, and provide reliable and reproducible results.

The invention proposes a solution to this need.

## SUMMARY

This invention thus relates to computer-implemented method for determining proportions $\alpha_i$ of C reference populations $P_i$ in an ensemble of biological objects being chosen among cells, vesicles of cellular origin, acellular microorganisms, and/or biofunctionalized materials, C being an integer number,

said ensemble of biological objects being associated with a set of test data,
the method comprising:

- receiving at least one set of reference data associated with a reference ensemble of biological objects,

wherein each reference data is labelled with a label $L_i$ belonging to a plurality of C labels $L_j$, each label $L_j$ among the plurality of C labels $L_j$ being associated with one corresponding reference population $P_j$ among the C reference populations,

- determining said proportions $\alpha_i$ by minimizing a distance between a first vector and a second vector,

said first vector being a weighted sum of C transformed vectors $z(\hat{P}_l)$ for i an integer varying from 1 to C, where $\hat{P}_l$ represents a matrix with m rows comprising a sample composed of the reference data labelled with the label $L_i$, z denotes a predetermined function, and the weight of each transformed vector

$z(\hat{P}_l)$ is the proportion $\alpha_i$,

said second vector resulting from the application of the predetermined function z to a second matrix Q comprising a sample composed of the test data.

The proposed advantageously method allows for quick quantification of populations in an ensemble of biological objects starting from labelled data, with input cytometric data and by only performing a minimization.

Advantageously, minimizing the distance between the first vector and the second vector comprises minimizing a quantity D defined by:

$$D = \frac{1}{2}\alpha^T P \alpha + q^T \alpha,$$

wherein $\alpha$ represents a vector comprising the proportions $\alpha_i$,

$\frac{1}{2}P = P'^T P'$, where P' is a matrix composed of the C vectors $z(\hat{P}_l)$,

$$q = -2\,P'^T z(Q).$$

In some embodiments, the predetermined function z is based on a kernel function k.

In some embodiments, computing P involves a computation of nxn values $k(x_{il}, x_{jt})$, wherein $x_{il}$ denotes an $i^{th}$ vector with the label $L_l$ in a matrix $P_0$ and $x_{jt}$ denotes a $j^{th}$ vector with the label $L_t$ of the matrix $P_0$, the matrix $P_0$ being a concatenation of the matrices $\hat{P}_l$, n being equal to $\sum_{b=1}^{C} n_b$, and, Q comprising m rows $y_j$, computing q involves a computation of n x m values $k(x_i, y_j)$.

Advantageously, the kernel function k is a Gaussian function.

In some other embodiments, the predetermined function is a vectorial function taking values in $\mathbb{R}^M$ and defined,

for a given vector x in $\mathbb{R}^S$ by:

$$z(x) = \sqrt{\frac{2}{M}}\left[\cos(\omega_i^T x)\,,\sin(\omega_i^T x)\right]_{i=1}^{M/2},$$

and for a given matrix X comprising r rows denoted $x_1, x_2,...x_r$, by:

$$z(X) = \frac{1}{r}\sum_{i=1}^{r} z(x_i),$$

where $(\omega i)_i \in {}_{1 .. M/2}$ is a statistical sample of a distribution $\Lambda_k$, $\Lambda_k$ being the Fourier transform of a translation invariant kernel function k. In those embodiments, the number of calculations performed in the method is advantageously reduced. Therefore, as a consequence, the computation time is reduced in comparison to prior art methods, as will be illustrated further. Furthermore, in comparison to machine learning models, no hyperparameter is needed. Also, when two sets of test data have to be compared, once the C transformed vectors $z(\hat{P}_l)$ and the second vector have been computed, the computation of an Euclidean distance is sufficient to perform the comparison, as compared to prior art methods as will be illustrated further. The comparison is therefore simpler, and thus faster.

Advantageously, the kernel function k is a Gaussian function. Having a kerner function k which is a Gaussian allows for easy computation of the distribution $\Lambda_k$.

In some embodiments, the method further comprises:

- receiving a set of K clusters $G_k$ associated with the set of test data, k being integer varying from 1 to K, and $G_k$ denoting the k-th cluster,

wherein each cluster $G_k$ in the set of K clusters $G_k$ is associated with a population and is represented with a matrix $X_k$,

- computing, for each cluster $G_k$ and for each vector $\hat{P}_l$, a distance d_ki between $z(X_k)$ and $z(\hat{P}_l)$,
- determining, for each cluster Gk, the reference population $Pp_k$ for which the distance dkp is minimal, wherein the reference population $Pp_k$ for is associated with the label $Lp_k$,
- assigning the label $Lp_k$ to the k-th cluster $G_k$. Therefore, the method advantageously allows for match labeling. In other words, the method allows, for instance, using the predetermined function defined as a vectorial function previously described, to label unlabelled clusters obtained from a segmentation method of cytometric data of an ensemble of biological objects.

In some embodiments, the biological objects are:

- cells of animal, plant, fungal, protist, bacterial or archaebacterial origin,
- vesicles of cellular origin chosen from exosomes, ectosomes, microvesicles, microparticles, prostasomes, oncosomes, matrix/calcification vesicles or apoptotic bodies,
- acellular microorganisms chosen from viruses, viroids and prions, and/or

- biofunctionalized materials comprising a material of synthetic or biological origin chosen from a nanoparticle (such as a nanobead, a nanosphere or a nanocapsule), a microparticle (such as a microbead, a microsphere or a microcapsule), a lipid vesicle (such as a unilamellar vesicle, a multilamellar vesicle, a lipoplex, a polyplex, a lipopolyplex, a liposome, a niosome, a cochleate, a virosome, an immunostimulating complex (ISCOM®)), said material of synthetic or biological origin being coupled to, or coated with, one or more peptide(s), protein(s), antibody(ies), antibody fragment(s), receptor(s), cytokine(s), chemokine(s), toxin(s), oligonucleotide(s), colored or fluorescent molecule(s), amine, carboxyl or hydroxyl group(s), bioactive molecule(s) (such as an immunomodulatory molecule, a small chemical molecule, a peptido-mimetic, a drug), molecule(s) of biotin, avidin or streptavidin, or a combination thereof.

Advantageously, the reference and test data are obtained by flow cytometry (or FACS for "fluorescence activated cell sorting"), by PCR-activated cell sorting (PACS), by microsphere affinity proteomics (MAP), by mass spectrometry, by chromatography, by CYTOF, by spectral cytometry, by mass cytometry, by image cytometry, by gene expression on chips (microarray), by sequencing (for example DNA-seq, RNA-seq, scDNA-seq, or scRNA-seq), by in situ hybridization, and/or by microscopy.

Advantageously, the reference and test data are obtained by single-cell analysis technologies or spatial biology analysis technologies.

Another aspect of the invention pertains to a device comprising a processor configured to carry out the method previously described.

Another aspect of the invention pertains to a computer program product comprising instructions which, when the program is executed by a computer, cause the computer to carry out the method previously described.

Another aspect of the invention relates to a non-transitory computer-readable recording medium comprising instructions which, when executed by a computer, cause the computer to carry out steps of the method previously described.

**DEFINITIONS**

In the present invention, the following terms have the following meanings:

The term "**processor**" or "**processing unit**" should not be construed as being limited to hardware capable of executing software, and refers generally to a processing device, which may include, for example, a computer, microprocessor, integrated circuit, or programmable logic device (PLD). The processor may also include one or more graphics processing units (GPUs), whether they are used for computer graphics and image processing or other functions. In addition, the instructions and/or data for executing the associated and/or resulting functionality may be stored on any media readable by the processor such as, for example, an integrated circuit, a hard disk, a CD (Compact Disc), an optical disk such as a DVD (Digital Versatile Disc), a RAM (Random-Access Memory) or a ROM (Read-Only Memory). In particular, instructions may be stored in hardware, software, firmware or any combination thereof.

"**Cytometry**" generally refers to the detection and/or measurement of characteristics of a cell, which is the basic structural, functional and biological unit of living organisms. By extension, cytometry also refers to the detection and/or measurement of characteristics of vesicles of cellular origin (such as extracellular or intracellular vesicles), or of particles of nano- or micrometer size such as acellular microorganisms (e.g., viruses), or biofunctionalized materials (e.g., microspheres coated with biological molecules such as proteins, or microspheres coated with bio-active molecules such as immunomodulatory molecules or drugs).

The "**cells**" may come from a unicellular or pluricellular organism, of prokaryotic or eukaryotic origin, of animal, vegetable, fungal, protist, bacterial or archaebacterial origin. They can be living, dead or fixed. The cells may, for example, come from solid or liquid tissues (such as bone marrow, blood or lymph, etc), or from body fluids (such as cerebrospinal fluid, urine, bronchoalveolar fluid, etc).

During the cytometric data acquisition process, the cells, vesicles or particles may be in suspension (aqueous or non-aqueous, biological or synthetic suspension), or immobilized on a solid support (e.g., a flask, a cell culture dish with one or more wells, a plate or microplate, a glass slide, a chip, etc.). They may have been pre-treated with biological or chemical agent(s).

The cells, vesicles or particles to be analyzed may be comprised within one or more populations.

A "**population**" is an ensemble of cells, vesicles, or particles with same or similar characteristics. A population can be subdivided into different subpopulations with secondary characteristics different from each other.

"**Cell-derived vesicles**" are vesicles composed of a membrane of cellular origin. It can be intracellular or extracellular vesicles. Extracellular vesicles have generally been secreted or excreted by a cell. "Cell-derived vesicles" include exosomes, ectosomes, microvesicles, microparticles, prostasomes, oncosomes, matrix/calcification vesicles, apoptotic bodies and other subsets of cellular vesicles.

"**Acellular microorganisms**" designate organisms of microscopic size whose structure is not cellular. "Acellular microorganisms" include viruses, viroids and prions.

The "**bio-functionalized materials**" or "**functionalized biomaterials**" or "bio-functionalized devices" or "bio-functionalized systems" designate any type of object comprising a material of synthetic or organic origin coated on its surface, coupled or linked to one or more molecule(s) or functional group(s) of biological origin.

As non-limitative examples, the material of synthetic or biological origin can be a nanoparticle (such as a nanobead, a nanosphere or a nanocapsule), a microparticle (such as a microbead, a microsphere or a microcapsule), a lipid vesicle such as a unilamellar vesicle, a multilamellar vesicle, a lipoplex, a polyplex, a lipopolyplex a liposome, a niosome, a cochleate, a virosome, an immune-stimulating complex (ISCOM®), etc.

Nanoparticles and microparticles, such as beads, spheres or capsules, can be organic, inorganic, magnetic or radioactive. For example, nanoparticles or microparticles can be made of metal, silica, aluminium, titanium, glass, ceramic, polystyrene, poly(methyl methacrylate), melamine, polylactide, latex, dextran, oxide, graphene, magnetic material, radioactive material, or a combination thereof.

As non-limited examples, to be bio-functionalized, the material can be combined with, or coated with, one or more peptide(s), protein(s), antibody(ies), fragment(s) of antibody, receptor(s), cytokine(s), chemokine(s), toxin(s), oligonucleotide(s), colored or fluorescent molecule(s), amine, carboxyl or hydroxyl group(s), bioactive molecule(s) (such as an immunomodulatory molecule, a small chemical molecule, a peptido-mimetic, a drug), biotin, avidin or streptavidin molecule(s), or a combination thereof.

In the context of the present invention, the term "**cytometric**", when qualifying data, parameters, measurements, events, may refer to cells, vesicles of cellular origin, or to particles such as acellular microorganisms or bio-functionalized materials.

A "**statistical sample**" refers to a subset of items from within a statistical population to estimate characteristics of the whole statistical population.

## BRIEF DESCRIPTION OF THE DRAWINGS

Figure 1 illustrates a device for determining proportions $\alpha_i$ of C reference populations $P_i$ in an ensemble of biological objects.

Figure 2 is an example of a flow-chart representing a method for determining proportions $\alpha_i$ of C reference populations $P_i$ in an ensemble of biological objects.

Figure 3 shows a comparison of performances of the method illustrated in Figure 2 and a method for quantification of biological objects of the prior art.

Figure 4 is an example of a flow-chart representing a method for match labeling.

## DETAILED DESCRIPTION

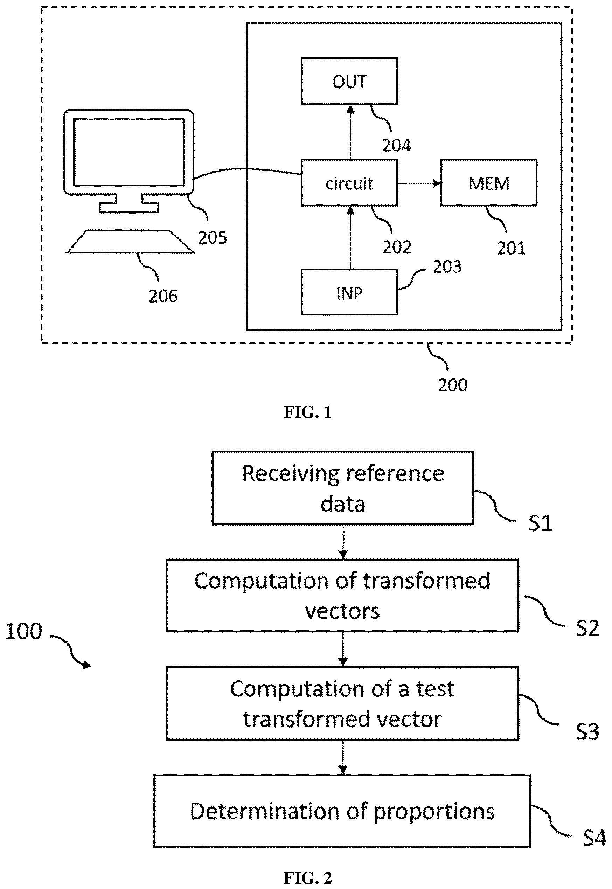

This invention relates to a computer-implemented method 100 (see figure 2) for determining proportions $\alpha_i$ of C reference populations $P_i$ in an ensemble of biological objects being chosen among cells, vesicles of cellular origin, acellular microorganisms, and/or biofunctionalized materials.

For instance, the biological objects are:

- cells of animal, plant, fungal, protist, bacterial or archaebacterial origin,
- vesicles of cellular origin chosen from exosomes, ectosomes, microvesicles, microparticles, prostasomes, oncosomes, matrix/calcification vesicles or apoptotic bodies,
- acellular microorganisms chosen from viruses, viroids and prions, and/or
- biofunctionalized materials comprising a material of synthetic or biological origin chosen from a nanoparticle (such as a nanobead, a nanosphere or a nanocapsule), a microparticle (such as a microbead, a microsphere or a microcapsule), a lipid vesicle (such as a unilamellar vesicle, a multilamellar vesicle, a lipoplex, a polyplex, a lipopolyplex, a liposome, a niosome, a cochleate, a virosome, an immunostimulating complex (ISCOM®)), said material of synthetic or biological origin being coupled to, or coated with, one or more peptide(s), protein(s), antibody(ies), antibody fragment(s), receptor(s), cytokine(s), chemokine(s), toxin(s), oligonucleotide(s), colored or fluorescent molecule(s), amine, carboxyl or hydroxyl group(s), bioactive molecule(s) (such as an immunomodulatory molecule, a small chemical molecule, a peptido-mimetic, a drug), molecule(s) of biotin, avidin or streptavidin, or a combination thereof.

Cytometric data can be obtained by any process allowing to analyze, determine or measure characteristics of a cell, vesicle or particle. The analysis can relate to a sample including a plurality of cells, vesicles and/or particles. For example, cytometric data can be obtained from a biological sample of an individual, such as one or more organ(s), tissue(s), cell(s) or cell fragment(s) of the individual.

In some cases, the cytometric data acquisition process may be preceded by a sample preparation process, including for example a stage of isolation of cells, vesicles and/or particles, or of preliminary isolation of components of said cells.

The sample preparation process comprising the biological objects with which cytometric data is associated may include, as non-limiting examples, one or more step(s) of magnetic-activated cell sorting (MACS), laser capture microdissection (LCM), manual cell or micro-manipulation harvesting, micro-fluidic, limit dilution, separation by optical tools, electrophoresis, separation by beads, immunoprecipitation, immunopanning, labeling, immunostaining, immunofluorescence, multiplexing and/or use of biochips.

Cytometric data can be data of "omics" or "meta-omics" technologies, for example genomics, epigenomics, transcriptomics, proteomics, metabolomics, lipidomics, etc. Thus, cytometric data can for example be relating to the detection and/or quantification of DNA (for example genes), RNA, proteins (for example cytokines, receptors, etc.), sugars, amino acids and/or fatty acids present in or on the surface of the cell, vesicle or particle, or any other molecule contained in the cell, vesicle or particle. Cytometric data can also be relating to the detection of the presence or absence of a particular molecule or a particular assembly in or on the surface of the cell, vesicle or particle, or to the detection of interactions between molecules present in or on the surface of the cell, vesicle or particle, or to the detection of interactions between cells and/or objects such as vesicles, microorganisms, or bio-functionalized materials.

By way of non-limitative and purely illustrative examples, when cytometric data are of the proteomic type, the cytometric parameters measured or analyzed can be the level of expression of one or more intracellular or extracellular protein(s), or the level of expression of one or more receptor(s) or marker(s) on the surface of the cell, vesicle or particle.

Other examples of cytometric parameters include the size of cells, vesicles or particles, their density, their granularity, their morphology/form, their refractive index, the composition of their membrane, their molecular content (such as for example the presence or the absence of an intracellular or surface molecule) or their content in this molecule (such as intracellular content, or ions, DNA, or RNA content ...) or the state of oxydo-reduction of the cell, its status in the cell cycle, its apoptotic state, or its level of phosphorylation ...

For instance, the cytometric data associated to the ensemble of biological objects may be obtained by flow cytometry (or FACS for "fluorescence activated cell sorting"), by PCR-activated cell sorting (PACS), by microsphere affinity proteomics (MAP), by mass spectrometry, by chromatography, by CYTOF, by spectral cytometry, by mass cytometry, by image cytometry, by gene expression on chips (microarray), by sequencing (for example DNA-seq, RNA-seq, scDNA-seq, or scRNA-seq), by in situ hybridization, and/or by microscopy. The term "microscopy" notably designates atomic force microscopy (AFM), electrochemical detection (EC), scanning electron microscopy (SEM), transmission electron microscopy (TEM), surface plasmon resonance (SPR), Raman micro-spectroscopy, etc.

The cytometric data can also be obtained by combining several of these techniques. By way of purely illustrative example, multiplexed profiling of RNA and protein expression at the single cell level can be obtained simultaneously by combining the PLAYR (Proximity Ligation Assay for RNA) technique, which makes it possible to measure the expression level of a large number of RNAs by flow or mass cytometry, with a technique for detecting surface or internal proteins labeled with antibodies.

In some embodiments, the cytometric data are obtained by single-cell analysis technologies. In some embodiments, the cytometric data are obtained by spatial biology analysis technologies, such as e.g. spatial genomics, transcriptomics or proteomics analysis technologies.

For example, the cytometric data may be obtained by single cell imaging-based technologies, such as e.g. flow, spectral or mass cytometry, or microscopy; or by sequencing-based technologies, such as e.g. single cell sequencing platform including for instance single-cell RNAseq, spatial transcriptomics, single-cell CITE-seq...

Another purely illustrative example of a technique for obtaining cytometric data is the profiling of single cells isolated in liquid droplets, enveloped by a thin semipermeable membrane (microcapsules), by high-throughput RNA cytometry, for example by multiplexed RT-PCR.

The analysis of the ensemble of biological objects outputted a set of test data 10. The set of test data 10 comprises statistical data about individual cell populations. The set of test data 10 will be sometimes referred to as "test statistical sample", where the expression "statistical sample" has been defined in the Definitions section. For instance, the set of test data 10 is stored in a matrix Q. The matrix Q comprises rows, each row corresponding to one biological object, and columns, each column corresponding to a characteristic of the corresponding biological object.

The method 100 may be implemented with a device 200 such as illustrated on Figure 1.

Figure 1 illustrates a device 200 for determining proportions $\alpha_i$ of C reference populations $P_i$ in an ensemble of biological objects according to embodiments of the invention.

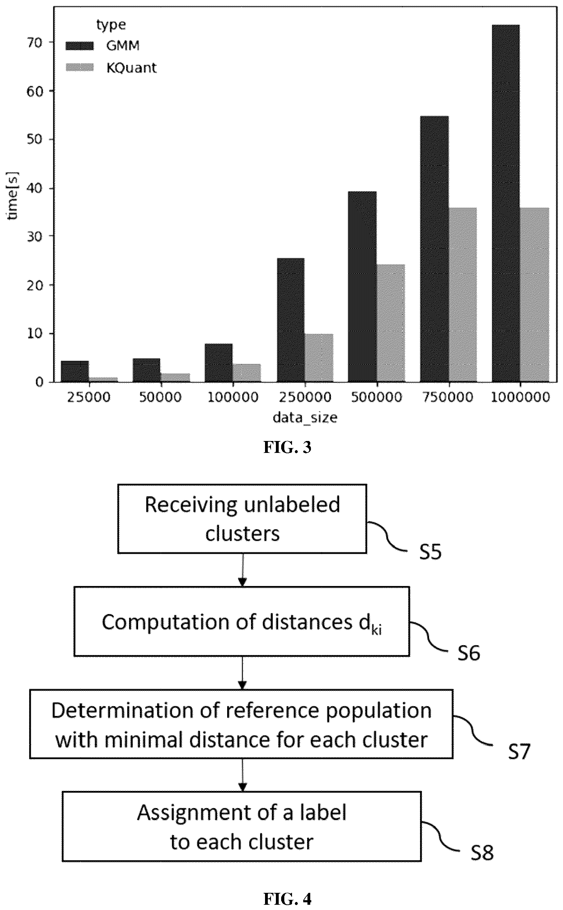

In these embodiments, the device 200 comprises a computer, this computer comprising a memory 201 to store program instructions loadable into a circuit and adapted to cause a circuit 202 to carry out steps of the methods of **Figures 2** and **3** when the program instructions are run by the circuit 202. The memory 201 may also store data and useful information for carrying steps of the present invention as described above.

The circuit 202 may be for instance:

- a processor or a processing unit adapted to interpret instructions in a computer language, the processor or the processing unit may comprise, may be associated with or be attached to a memory comprising the instructions, or
- the association of a processor / processing unit and a memory, the processor or the processing unit adapted to interpret instructions in a computer language, the memory comprising said instructions, or
- an electronic card wherein the steps of the invention are described within silicon, or
- a programmable electronic chip such as a FPGA chip (for « Field-Programmable Gate Array »).

The computer may also comprise an input interface 203 for the reception of input data and an output interface 204 to provide output data. Examples of input data and output data will be provided further.

To ease the interaction with the computer, a screen 205 and a keyboard 206 may be provided and connected to the computer circuit 202.

A database comprising at least one set of reference data is preliminarily collected.

In some embodiments, the at least one set of reference data comprises N sets of reference data, with N being an integer number higher than or equal to 1. Each set of reference data comprises cytometric data corresponding to a given reference ensemble of biological objects. For instance, the cytometric data associated to a plurality of given reference biological objects have been obtained by flow cytometry (or FACS for "fluorescence activated cell sorting"), by PCR-activated cell sorting (PACS), by microsphere affinity proteomics (MAP), by mass spectrometry, by chromatography, by CYTOF, by spectral cytometry, by mass cytometry, by image cytometry, by gene expression on chips (microarray), by sequencing (for example DNA-seq, RNA-seq, scDNA-seq, or scRNA-seq), by in situ hybridization, and/or by microscopy.

Each given reference ensemble of biological objects originates from one among the given reference ensemble of biological objects, each of which containing different reference populations $D_i$ of biological objects. Each reference population $D_i$ is assigned a label $L_i$ belonging to a plurality of C labels $L_j$, C being an integer number higher than or equal to 2 and j varying from 1 to C. The cytometric data of each set of reference data will often be referred to as "reference statistical sample", where the expression "statistical sample" has been defined in the Definitions section.

In those embodiments, each reference data in a set of reference data is labelled with a label $L_j$ belonging to a plurality of C labels $L_j$, j being an integer varying between 1 and C.

In some embodiments, the data contained in the database take the form of an electronic file comprising C matrices $\hat{P}_J$. Each matrix $\hat{P}_J$ contains a concatenation of the reference data labelled with the label $L_j$ in the N sets of reference data. The higher the number N of sets of reference data, the more precise the result of the quantification obtained with the method 100 will be. For instance, each matrix $\hat{P}_J$ comprises $n_j$ rows, each row corresponding to data pertaining to a biological object. All biological objects represented in the matrix $\hat{P}_J$ present the label $L_j$. The electronic file can be received by the device 200 via the input interface 203 and subsequently stored in the memory 201 of the device 200.

The computer-implemented method 100 aims at determining the proportions $\alpha_j$ of each reference population $D_j$ among the C reference populations $D_j$, j being an integer varying between 1 and C. The proportions $\alpha_j$ can be gathered in a single vector $\alpha$.

Figure 2 is a flowchart illustrating an example of steps to be executed to implement the computer-implemented method 100.

In a step S1, the at least one set of reference data is received. As explained above, the at least one set of reference data can take the form of an electronic file comprising C matrices $\hat{P}_J$ as previously described.

In a step S2, transformed vectors $M_j$ are computed. The transformed vectors result from the application of a predetermined function z on the matrices $\hat{P}_J$. The predetermined function z will be described further. In other words, a transformed vector $M_j$ is defined by: $M_j = z(\hat{P}_J)$.

In a step S3, a transformed vector R, referred to as test transformed vector, is computed. The test transformed vector R results from the application of the predetermined function z on a matrix Q comprising the set of test data 10 as previously described. In other words, the test transformed vector R is defined by:

$$R = z(Q)$$

In a step S4, the proportions $\alpha_j$, for j varying between 1 and C, are determined by minimizing a distance between the weighted sum of the transformed vectors $\sum_{i=1}^{C} \alpha_i z(\hat{P}_i)$ and the test transformed vector R.

Steps S1 to S4 may be implemented by the processor of the circuit 202. Advantageously, the processor includes one or more graphics processing units (GPUs).

In some embodiments, step S4 is implemented by minimizing the quantity D defined by:

$$D = \frac{1}{2}\alpha^T P \alpha + q^T \alpha,$$

where:

$\frac{1}{2}P = P'^T P'$, P' representing a matrix composed of the C vectors $z(\hat{P}_l)$,

$q = -2 P'^T z(Q)$. The expression $P'^T$ denotes the transposed matrix of the matrix P'. The minimization is carried out under the conditions that $\sum_{i=1}^{C} \alpha_i$ is equal to 1 and all the proportions $\alpha_i$ are positive.

In some embodiments referred to as kernel embodiments, the predetermined function z is a kernel function k, such as functions defined with respect to reproducing kernel Hilbert spaces (RKHS) and used in techniques known as kernel methods. In those embodiments, the concept of kernel mean embedding is used. For instance, the kernel function k is Gaussian function. We introduce a matrix $P_0$, which is the concatenation of the matrices $\hat{P}_J$. Each matrix $\hat{P}_J$, for j taking values between 1 and C, comprises $n_j$ rows. In this case, the matrix $P_0$ comprises n rows, where n is equal to the sum of the numbers $n_j$ for j comprised between 1 and C. The rows of the matrix $P_0$ are denoted $x_{il}$, where i denotes the $i^{th}$ row of the matrix $\hat{P}_l$ included in the matrix $P_0$. It can be shown that the computation of the quantity P in the definition of the quantity D involves the computation of nxn values $k(x_{il},x_{jt})$. Similarly, we assume that the matrix Q comprises m rows $y_i$. In this case, it can be shown that the computation of the quantity q used in the definition of the quantity D involves a computation of n x m values $k(x_{il},y_j)$.

In some other embodiments referred to as vectorization embodiments, the predetermined function z is a vectorial function taking values in $\mathbb{R}^M$ and defined, for a given vector x in $\mathbb{R}^S$ by:

$$z(x) = \sqrt{\frac{2}{M}} \left[\cos(\omega_i^T x), \sin(\omega_i^T x)\right]_{i=1}^{M/2},$$

and for a given matrix X comprising r rows denoted $x_1, x_2,...x_r$, by:

$$z(X) = \frac{1}{r}\sum_{i=1}^{r} z(x_i),$$

where $(\omega i)_{i \in 1 .. M/2}$ is a statistical sample of a distribution $\Lambda_k$, $\Lambda_k$ being the Fourier transform of a translation invariant kernel function k. For instance, the translation invariant kernel function k is a Gaussian function.

In those embodiments, the concept of Random Fourier feature matching is used.

In the vectorization embodiments, step S4 is simpler as it can be shown that no further calculation, except for the minimization of the quantity D, is necessary. The computation of the transformed vectors $M_j$ ($z(\hat{P}_j)$), and of the test transformed vector R ($z(Q)$) is sufficient. In other words, to compute $z(\hat{P})$, the matrix containing the concatenated N sets of reference data is multiplied by the matrix containing the values of the statistical sample $(\omega_i)_{i \in 1 .. M/2}$ of the distribution $\Lambda_k$, Then, the cosine and the sine functions are applied to the results. For instance, to minimize the quantity D, a solver program can be used. An example of software package that can be used is CVXOPT.

This reduced number of computations provides one of the advantages of the method 100 in the vectorization embodiments, which is the computational speed, as compared to prior art methods.

Figure 3 shows a comparison of computation times between the method 100 (referred to on Figure 3 as KQuant) and a method for quantification of biological objects of the prior art referred to on Figure 3 as GMM and described in the paper "A Machine Learning Approach to the Classification of Acute Leukemias and Distinction From Nonneoplastic Cytopenias Using Flow Cytometric Data", by Monaghan S.A. and all, in American Society for Clinical Pathology, 2021. The method GMM is a machine learning based method for classifying multidimensional data obtained by flow cytometry. Both methods 100 and GMM were applied on a series of test data of increasing size (from 25000 points, i.e. biological objects in the test data, to 1,000,000 points). It can be observed that the computation time is shorter with the method 100 than with the method GMM from the prior art for all sizes.

Advantageously, in the vectorization embodiments, a specific library can be used. For instance, the KeOps library, that allows computing reductions of large arrays whose entries are given by a mathematical formula or a neural network, can be used. This library can be used with Python (NumPy, PyTorch), Matlab and R.

In some embodiments, referred to as match labeling embodiments, the method 100 further comprises additional steps S5, S6, S7 and S8, that will be described below, and allowing to perform match labeling.

Indeed, there exists some methods to segment a set of cytometric data into clusters corresponding to populations or subpopulations. However, often, those methods do not provide labels of the clusters obtained via the segmentation.

In the match labeling embodiments, the predetermined function z is the one as used in the vectorization embodiments previously described.

Figure 4 is a flowchart illustrating an example of further steps of the method 100 as illustrated in Figure 2 to be executed to implement a computer-implemented method for match labeling.

In a step S5, a set of clusters $G_k$ associated with the set of test data 10 is received. The set of clusters $G_k$ may have been preliminary obtained by the application of a segmentation method on the set of test data 10. For instance, the set of clusters $G_k$ are received via the input interface 203 of the device 200. By definition, each cluster $G_k$ in the set of clusters $G_k$ is associated with a population. Typically, each cluster $G_k$ is received in the form of a matrix $X_k$.

In a step S6, a distance $d_{ki}$ between $z(Xk)$ and $z(\hat{P}_l)$, for each cluster $G_k$ and for each vector $\hat{P}_l$.

In a step S7, for each cluster $G_k$, the reference population $Pp_k$ for which the distance $d\_kp_k$ is minimal is determined, $p_k$ being an integer comprised between 1 and C.

In a step S8, each label $Lp_k$ corresponding to

the reference population $Pp_k$ determined at step S7 is assigned to the corresponding cluster $G_k$.

Steps S6, S7 and S8 may be implemented by the processor of the circuit 201 of the device 201.

**Claims**

1. A computer-implemented method (100) for determining proportions $\alpha_i$ of C reference populations ($P_i$) in an ensemble of biological objects being chosen among cells, vesicles of cellular origin, acellular microorganisms, and/or biofunctionalized materials, C being an integer number,

   said ensemble of biological objects being associated with a set of test data (10), said method comprising:

   - receiving at least one set of reference data associated with a reference ensemble of biological objects,

   wherein each reference data is labelled with a label L; belonging to a plurality of C labels ($L_j$), each label ($L_j$) among the plurality of C labels ($L_j$) being associated with one corresponding reference population ($P_j$) among the C reference populations,

   - determining said proportions $\alpha_i$ by minimizing a distance between a first vector and a second vector,

   said first vector being a weighted sum of C transformed vectors $z(\hat{P}_l)$ for i an integer varying from 1 to C, where $\hat{P}_l$ represents a matrix with $n_i$ rows comprising a sample composed of the reference data labelled with the label ($L_i$), z denotes a predetermined function, and the weight of each transformed vector $z(\hat{P}_l)$ is the proportion $\alpha_i$, said second vector resulting from the application of said predetermined function z to a second matrix Q comprising a sample comprising the set of test data (10).

2. The method according to claim **1,** wherein minimizing the distance between the first vector and the second vector comprises minimizing a quantity D defined by:

$$D = \frac{1}{2}\alpha^T P \alpha + q^T \alpha,$$

   wherein $\alpha$ represents a vector having as coefficients the proportions $\alpha_i$,

$$\frac{1}{2}P = P'^T P'$$

, where P' is a matrix composed of the C vectors $z(\hat{P}_l)$,

$$q = -2\,P'^T z(Q).$$

3. The method according to claim **1** or **2,** wherein the predetermined function z is based on a kernel function k.

4. The method according to claim **3,** wherein,

   - computing P involves a computation of nxn values $k(x_{il},x_{jt})$, wherein $x_{il}$ denotes an $i^{th}$ vector with the label $L_l$ in a matrix $P_0$ and $x_{jt}$ denotes a $j^{th}$ vector with the label $L_t$ of said matrix $P_0$, the matrix $P_0$ being a concatenation of the matrices $\hat{P}_l$, n being equal to $\sum_{b=1}^{C} n_b$ ,
   - Q comprises m vectors $y_j$, and computing q involves a computation of n x m values $k(x_{il},y_j)$.

5. The method according to claim **3** or **4,** wherein the kernel function k is a Gaussian function.

6. The method according to claim **1** or **2,** wherein the predetermined function z is a vectorial function taking values in $\mathbb{R}^M$ and defined,

   for a given vector x in $\mathbb{R}^S$ by:

$$z(x) = \sqrt{\frac{2}{M}}\left[\cos(\omega_i^T x),\sin(\omega_i^T x)\right]_{i=1}^{M/2},$$

   and for a given matrix X comprising r rows denoted $x_1, x_2,...x_r$, by:

$$z(X) = \frac{1}{r}\sum_{i=1}^{r} z(x_i)\,,$$

   where $(\omega i)_{i \in 1 .. M/2}$ is a statistical sample of a distribution $\Lambda_k$, $\Lambda_k$ being the Fourier transform of a translation invariant kernel function k.

7. The method according to claim **6,** wherein the kernel function k is a Gaussian function.

8. The method according to claim **6** or **7,** further comprising:

   - receiving a set of K clusters ($G_k$) associated

with the set of test data (10), k being integer varying from 1 to K, and (Gk) denoting the k-th cluster,

wherein each cluster $(G_k)$ in the set of K clusters $(G_k)$ is associated with a population and is represented with a matrix $X_k$,

- computing, for each cluster $(G_k)$ and for each vector $\hat{P}_l$, a distance d_ki between $z(X_k)$ and $z(\hat{P}_l)$,
- determining, for each cluster $(G_k)$, the reference population $Pp_k$ for which the distance $d\_kp_k$ is minimal, wherein the reference population $Pp_k$ is associated with the label $(Lp_k)$,
- assigning the label $(Lp_k)$ to the k-th cluster $(G_k)$.

9. The method according to any one of claims **1** to **8,** wherein the biological objects are:

- cells of animal, plant, fungal, protist, bacterial or archaebacterial origin,
- vesicles of cellular origin chosen from exosomes, ectosomes, microvesicles, microparticles, prostasomes, oncosomes, matrix/calcification vesicles or apoptotic bodies,
- acellular microorganisms chosen from viruses, viroids and prions, and/or
- biofunctionalized materials comprising a material of synthetic or biological origin chosen from a nanoparticle (such as a nanobead, a nanosphere or a nanocapsule), a microparticle (such as a microbead, a microsphere or a microcapsule), a lipid vesicle (such as a unilamellar vesicle, a multilamellar vesicle, a lipoplex, a polyplex, a lipopolyplex, a liposome, a niosome, a cochleate, a virosome, an immunostimulating complex (ISCOM®)), said material of synthetic or biological origin being coupled to, or coated with, one or more peptide(s), protein(s), antibody(ies), antibody fragment(s), receptor(s), cytokine(s), chemokine(s), toxin(s), oligonucleotide(s), colored or fluorescent molecule(s), amine, carboxyl or hydroxyl group(s), bioactive molecule(s) (such as an immunomodulatory molecule, a small chemical molecule, a peptidomimetic, a drug), molecule(s) of biotin, avidin or streptavidin, or a combination thereof.

10. The method according to any one of claims **1** to **9,** wherein the reference and test data are obtained by flow cytometry (or FACS for "fluorescence activated cell sorting"), by PCR-activated cell sorting (PACS), by microsphere affinity proteomics (MAP), by mass spectrometry, by chromatography, by CYTOF, by spectral cytometry, by mass cytometry, by image cytometry, by gene expression on chips (microarray), by sequencing, by in situ hybridization, and/or by microscopy.

11. The method according to any one of claims **1** to **10,** wherein the reference and test data are obtained by single-cell analysis technologies or spatial biology analysis technologies.

12. A device comprising a processor configured to carry out a method according to any one of claims **1** to **11.**

13. A computer program product comprising instructions which, when the program is executed by a computer, cause the computer to carry out the method of any one of claims **1** to **11.**

**FIG. 1**

**FIG. 2**

**FIG. 3**

**FIG. 4**

Europäisches
Patentamt

European
Patent Office

Office européen
des brevets

## EUROPEAN SEARCH REPORT

**Application Number**

EP 23 30 5847

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MONAGHAN SARA A ET AL: "A Machine Learning Approach to the Classification of Acute Leukemias and Distinction From Nonneoplastic Cytopenias Using Flow Cytometry Data", AMERICAN JOURNAL OF CLINICAL PATHOLOGY, vol. 157, no. 4, 1 April 2022 (2022-04-01) , pages 546-553, XP093093510, US ISSN: 0002-9173, DOI: 10.1093/ajcp/aqab148 Retrieved from the Internet: URL:https://watermark.silverchair.com/aqab 148.pdf?token=AQECAHi208BE49Ooan9kkhW_Ercy 7Dm3ZL_9Cf3qfKAc485ysgAAA2cwggNjBgkqhkiG9w 0BBwaggNUMIIDUAIBADCCA0kGCSqGSIb3DQEHATAe BglghkgBZQMEAS4wEQQMxrOFrzBViwhB8boqAgEQgI IDGlrvTq23EmLaKekUG3B_5EE___sp81_fS0_eUTCUc zS9sMw_-6Gf4zlUJoB3vIvRCsf146OdWnUoK9AWDgu m-5IfwQlTj> * abstract * * page 548, column 1, paragraph 1 - page 549, column 1, paragraph 3; figure 1 * ----- | 1-13 | INV. G16B40/00 |
| A | Wikipedia: "Support vector machine", , 22 May 2023 (2023-05-22), pages 1-11, XP093093620, Retrieved from the Internet: URL:https://en.wikipedia.org/w/index.php?t itle=Support_vector_machine&oldid=11563322 83 [retrieved on 2023-10-20] * abstract * ----- | 1-11 | TECHNICAL FIELDS SEARCHED (IPC) G16B |

The present search report has been drawn up for all claims

| Place of search | Date of completion of the search | Examiner |
|---|---|---|
| Munich | 25 October 2023 | Schmitt, Constanze |

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MONAGHAN S.A.** A Machine Learning Approach to the Classification of Acute Leukemias and Distinction From Nonneoplastic Cytopenias Using Flow Cytometric Data. *American Society for Clinical Pathology,* 2021